# EUROPEAN PATENT APPLICATION

(11) **EP 2 248 467 A1**
(43) Date of publication of application: **10.11.2010**
(21) Application number: 09006081.5
(22) Date of filing: 04.05.2009
(51) Int. Cl.: A61B 6/04, A61G 13/02, A61G 13/08, A61B 17/225, A61B 19/00

(54) **Examination and therapy table**

(71) Applicant: Dornier MedTech Systems GmbH, 82234 Wessling (DE)
(72) Inventor: Artmeier, Theo, 82194 Gröbenzell (DE)
(74) Representative: Grünecker, Kinkeldey, Stockmair & Schwanhäusser Anwaltssozietät

(57) **Abstract**

The present invention relates to an examination and therapy table having an access region, a support region, and a patient supporting plate, the patient supporting plate comprising an examination portion having means for facilitating examination, and a therapy portion having means for facilitating therapy, wherein there is an examination configuration in which the examination portion of the patient supporting plate is arranged in the access region of the table, whereas the therapy portion of the patient supporting plate is arranged in the support region of the table. The invention has the object to provide an improved examination and therapy table with a better and simplified design to allow for simplified configuration procedures during use. This is solved by an examination and therapy table that is convertible into a therapy configuration in which the therapy portion of the patient supporting plate is arranged in the access region of the table, and the examination portion of the patient supporting plate is arranged in the support region of the table.

## Description

The present invention relates to an examination and therapy table with the features of the preamble of claim 1.

A variety of medical applications, in particular examination applications and therapy applications, involve radiography, alone or in combination with other treatment examination or technologies. One example is extracorporeal shock wave lithotripsy, which is a non-invasive treatment of, for instance, kidney stones. Said kidney stones are broken up using an acoustic pulse that is applied from the outside of a patient's body. In order to readily locate the kidney stones during treatment, a simultaneous radiography can be conducted, providing respective target information.

During examination or therapy, a patient usually lies flat on a therapy table. Such therapy tables can be equipped with means for facilitating therapy. In the field of lithotripsy, such means can be recesses, i.e. cut-outs in a patient supporting plate on which the patient lies during therapy. The cut-outs allow for easy access to the patient and are arranged in a therapy region of the patient supporting plate. The same device used for radiography during lithotripsy can usually be used for conventional radiographic examinations, as sometimes other radiographic devices are provided in the same room as the lithotripter. Lithotripsy is usually conducted by urologists and the radiographic devices are very often used for other radiographic examinations of the urogenital tract of a patient. For such examination procedures, a separate examination table or a radiolucent examination portion on the patient supporting plate is necessary to obtain satisfactory images since cut-outs can cause interferences on said images.

DE 199 15 852 C2 discloses a urological patient bed having a first plate section made of x-ray-transparent material, forming an examination area, and a second plate section forming a treatment area and having a lateral cut-out. The first and second plate sections and two further extending plate sections can be disassembled and reassembled again in a different configuration to allow for access to the patient from the opposing side. While the treatment area is mounted to a support, the first plate section can be attached to two opposing sides of the second table section in order to reconfigure the patient bed. This renders a considerable workload for medical personnel or auxiliary personnel to reconfigure said patient bed.

Other patient supporting tables are known in the art, which comprise exchangeable center sections having one or more cut-outs for lithotripsy applications or having no cut-out for endo-urological applications, which involve a radiographic examination of the patient's urinary tract. The exchangeable center sections that are momentarily not used are stored at a separate location.

It has been found that the patient tables known in the art render drawbacks due to their complicated design and complicated reconfiguring procedures.

It is an object of the present invention to provide an improved examination and therapy table with a better and simplified design to allow for simplified reconfiguration procedures during use.

The object is solved by an examination and therapy table, hereinafter referred to as "table", having the features of independent claim 1.

For examination, the table is provided in an examination configuration in which the therapy portion of the patient supporting plate is arranged in a support region of the table while the examination portion is arranged in the access region. The table is convertible into a therapy configuration in which the therapy portion of the patient supporting plate is arranged in the access region of the table, and the examination portion of the patient supporting plate is arranged in the support region of the table. The advantage is that it is easier to reconfigure the table and a presently "not-in-use" section is arranged in the support region.

In a preferred embodiment, the examination portion can be moveable in conjunction with the therapy portion from the examination configuration into the therapy configuration and vice versa. The advantage of this design is that few parts need to be operated to change the configuration of the table. Hence, the number of reconfiguration steps can be reduced, which considerably eases its use.

Advantageously, the access region can adjoin one lateral side of the support region. This allows for a simplified design of the entire table, and allows positioning the examination and/or therapy equipment together at one side of the table.

In preferable embodiments, the patient supporting plate may be rotatable between the examination configuration and the therapy configuration. Compared to the prior art, this renders a great advantage, as the patient supporting plate can just be turned around between the examination configuration and the therapy configuration. Less operation work is required, which renders positive effects in terms of required time and complexity of handling.

In a further embodiment, a center of rotation may be arranged approximately at a transition region between the access region and the support region of the examination and therapy table. Being provided with such a configuration, the patient supporting plate can just be turned around while rotating about the center of rotation in a space-saving manner.

In another favourable embodiment, the patient supporting plate may be rotatable by at least 180 degrees. This allows arranging the examination portion and the therapy portion in an opposing manner and to simply rotate the patient supporting plate from one configuration, for instance, the examining configuration, into the second configuration, for instance, the therapy configuration.

It may be preferable that the patient supporting plate rotates about an approximately vertical axis. This bears the advantage that the patient supporting plate can be rotated between the examination configuration and the therapy configuration in an approximately horizontal plane.

In a further preferable embodiment, the patient supporting plate may be rotatably connected to a supporting means of the examination and therapy table. By means of a rotatable connection between the patient supporting plate and the supporting means, which supporting means is preferably arranged within the support region, a guidance is provided so that a user just needs to move the patient supporting plate between the examination configuration and the therapy configuration and at least a large part of the weight of the whole patient supporting plate is borne by the supporting means. Further, the patient supporting plate can thereby be rotatable relative to the supporting means so that the patient supporting plate can be rotated in an easy manner. This immensely eases the use and alleviates the physical workload of the respective medical personnel.

Advantageously, the patient supporting plate may be rotatable about a central axis, preferably arranged approximately central relative to a width and/or a length of the patient supporting plate. The patient supporting plate rotates about its assumed center of axis, the area of the cut-outs considered as part of the area of the patient supporting plate, which allows for an easy to understand and predictable motion of the patient supporting plate relative to the rest of the table, which helps the medical personnel to remove possible obstacles within the circumference of the table prior to conversion of the table.

In one further preferred embodiment, the patient supporting plate may be supported by at least two, preferably three bearing means arranged in the support region of the examination and therapy table. This minimizes unwanted tilting and wobbling, as the bearing means firmly support the patient supporting plate.

In a particularly favourable embodiment, the patient supporting plate may be connected to a support structure via an articulated joint, the articulated joint being capable of receiving transverse forces approximately in X - Y directions and normal forces in a Z direction. The advantage of such a design is that a majority of the loads exerted on the patient supporting plate is received by one structure so that minor efforts are required to compensate for additional loads, like torques.

In an advantageous embodiment, the patient supporting plate may be connected to a supporting means of the examination and therapy table via a shaft structure. A shaft structure allows for easy rotation and firm support of transverse forces, which might occur in plane with the patient supporting plate. Moreover, shaft structures require little maintenance.

In a preferred embodiment, at least one locking means may be provided that locks the patient supporting plate in at least the examination configuration and/or the therapy configuration, preferably against a rotation. It was found that locking means are a good way to safely retain the patient supporting plate against inadvertent motions of the patient supporting plate during an examination or therapy procedure, especially when motions of the patient supporting plate are apt to cause threats to a patient during the use of radiographic devices or lithotripsy devices. The locking means can incorporate one or more of the bearing means, as described above.

In a more preferable embodiment, the locking means may be releasable. Thereby, medical personnel can easily release the locking of the patient supporting plate in order to contribute to an easy reconfiguration of the examination and therapy table.

In one further embodiment, the locking means may comprise clamping means to clamp at least one portion of the patient supporting plate to a support structure of the examination and therapy table. Such means facilitate a good and safe locking of the patient supporting plate to the examination and therapy table, and can easily compensate for torque loads induced by loads exerted on projecting portions of the patient supporting plate.

Preferably, the locking means may comprise means for securing the patient supporting plate against rotation about an axis, approximately about a Z direction. Thereby, the patient supporting plate can easily be prevented from rotating in its X-Y plane, while intended other rotations might still be possible. Such intended rotations can include, for example, the Trendelenburg rotation.

It is possible that a locking means may be provided at the end of the patient supporting plate that is arranged in the support region. Such a structure allows that most of the functional and moveable parts are disposed separate to the access region of the table so as not to interfere with respective examination or therapy applications or devices.

Advantageously, a column may be arranged at a central position of the support region. A central column is easily capable of carrying loads exerted on top of the examination and therapy table while incorporating a simple and effective design.

Particularly advantageously, a center of rotation and/or a locking means can be arranged at opposing lateral sides of an X-Y positioning unit, wherein the X-Y positioning unit is arranged between the patient supporting plate and the supporting structure. Providing an X-Y positioning unit between the patient supporting plate and the supporting structure of the table allows that the patient supporting plate is moveable in conjunction with the patient lying thereon relative to the supporting structure. While maintaining the respective examination or therapy configuration, such a movement is limited to a range of several centimeters in each direction. Thereby, for instance, during radiography, the patient can be transferred in relation to the radiography device in order to expand the imaging region. The X-Y positioning unit can also be used for more easily fine-positioning the patient relative to the examination or treatment devices.

Favourably, the patient supporting plate may integrally incorporate at least the examination portion and the therapy portion. This can simplify the manufacture of the patient supporting plate, i.e. a one-piece carbon fibre patient supporting plate is a possible alternative.

Preferably, the examination portion may be homogeneous. As the examination portion is optimized to facilitate examination, preferably by radiography, a homogeneous structure can drastically reduce shadings and interferences on radiographic images. A portion of the patient supporting plate is also characterized as "homogeneous", having a constant thickness and/or minimal or no underlying mechanical structures, like supporting bars or cut-outs. This "homogeneous" property basically renders a constant shading of radiography throughout the entire area.

Particularly advantageous, the therapy portion of the patient supporting plate may be permeable for radiography. Such a structure allows that therapy applications can be supplemented by a simultaneous radiographic examination.

The features described above in connection with diverse embodiments may be combined to form further preferable embodiments.

One exemplary embodiment of the invention is shown in the drawings, and is described hereinafter. The following is shown in the drawings:
- Figure 1: shows a schematic perspective view of the inventive examination and therapy table assuming an examination configuration;
- Figure 2: shows a schematic perspective view of the inventive examination and therapy table assuming a therapy configuration;
- Figure 3: shows a schematic side view of the examination and therapy table assuming the therapy configuration;
- Figure 4: shows a schematic top view of the examination and therapy table assuming the therapy configuration;
- Figure 5: shows a schematic top view of the examination and therapy table assuming the examination configuration.

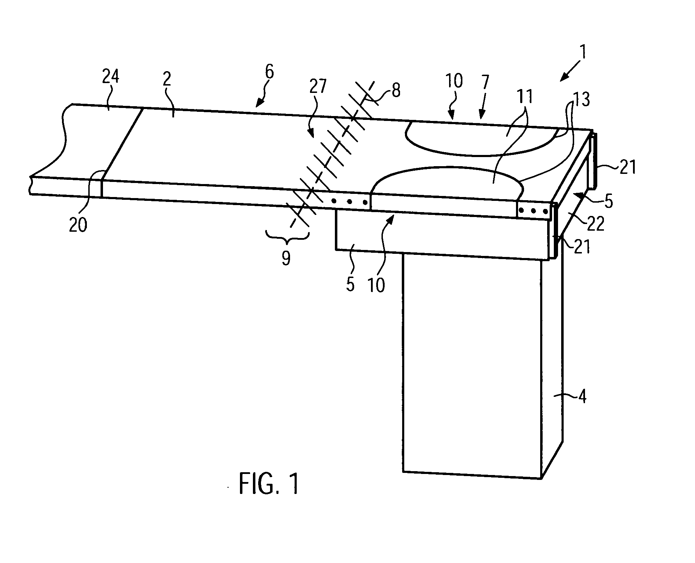

Figure 1 shows the examination and therapy table 1 having a patient supporting plate 2 on top. The patient supporting plate 2 comprises an examination portion 6 and a therapy portion 7. The examination portion 6 has means for facilitating examination and can be functionally and structurally delimited from the therapy portion 7 approximately at the delimiting broken line 8. However, the delimiting broken line 8 can also be understood as showing the center of a transition region 9 between the examination portion 6 and the therapy portion 7.

The therapy portion 7 opposes the examination portion 6 relative to the delimiting dashed line 8. The means for facilitating examination in the present example is that the examination portion 6 is formed as a radiolucent section that is homogeneous. As this portion of the table is optimized for radiography, it is called the examination portion 6.

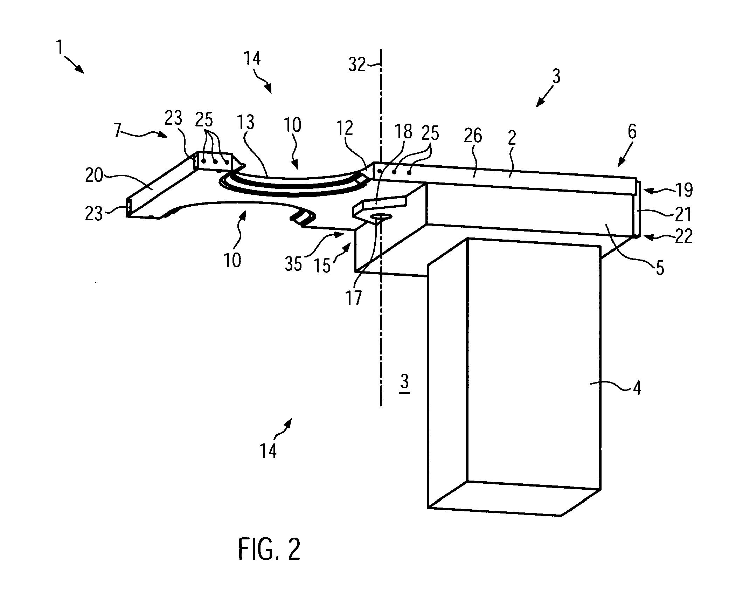

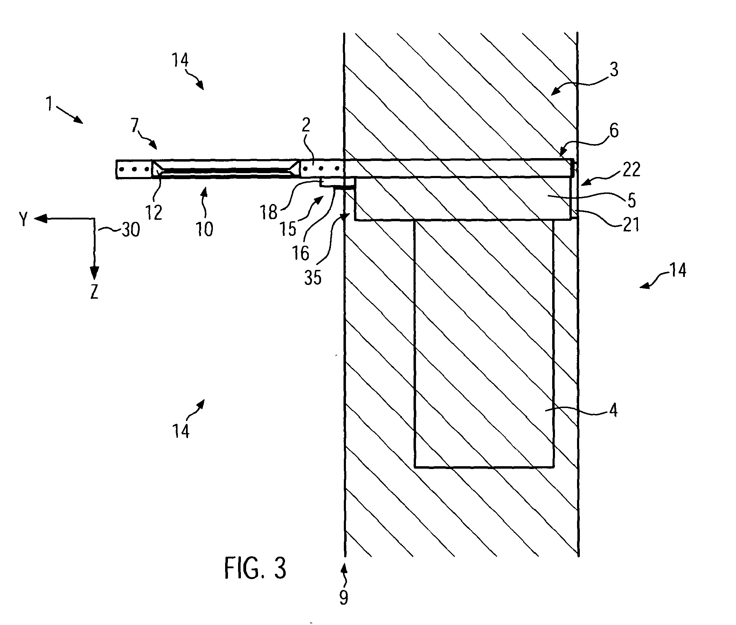

In the context of radiography, the meaning of homogeneous is that the radiographed structure is formed such that a shading on a radiographic image caused by the x-rays penetrating the patient supporting plate 2 is constant. This is implemented by special mechanical properties and suitable materials. As can be seen from the side view of Figure 3 and in the perspective view of Figure 2, cut-out 10 comprises a ridge portion 12 having a somewhat triangular cross-section and extending along arcuate edges 13 of cut-outs 11. As the thickness of the patient supporting plate 2 is divergent at ridge portion 12, it is inhomogeneous so that the shading caused by ridge portion 12 on a radiographic image is not constant and thus image interference, that is, inhomogeneous shading, appears on the radiographic image. The examination portion 6 has an approximately constant thickness, comprises no interfering structure and is permeable for radiography, so that it is considered to be homogeneous.

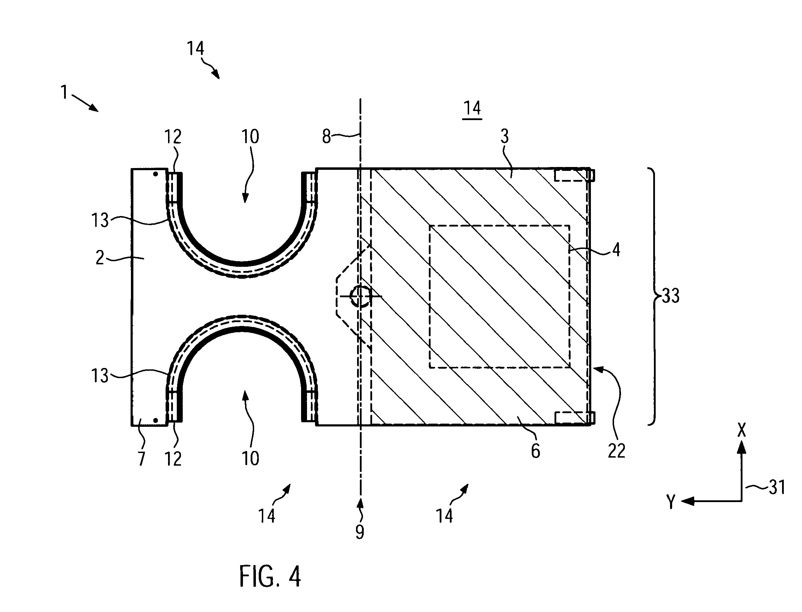

The therapy portion 7 comprises means for facilitating therapy, which are incorporated by cut-outs 10 and inserts 11 that fit in the cut-outs 10. By removing one or both of the inserts 11, as shown in Figures 2 and 4, a patient lying on top of the patient supporting plate becomes accessible, for instance, for a C-arm of a lithotripter during a therapy application in which the table assumes the therapy configuration, also shown in Figures 2 to 4.

Similar to the examination portion, the therapy portion 7 is also permeable for radiography or radiolucent so that radiography can be used in combination with therapy appliances like lithotripters that require the means for facilitating therapy, that is cut-outs 10.

Figures 3 to 5 show hatched areas that circumscribe the support region 3 of the examination and therapy table 1. Said figures indicate that the support region 3 spans a somewhat cuboid volume. Basically, the support region 3 is the region of the examination and therapy table 1 within which the loads exerted on the patient supporting plate 2 are supported and passed to a supporting means and then into the floor on which the supporting means of the examination and therapy table 1 rests.

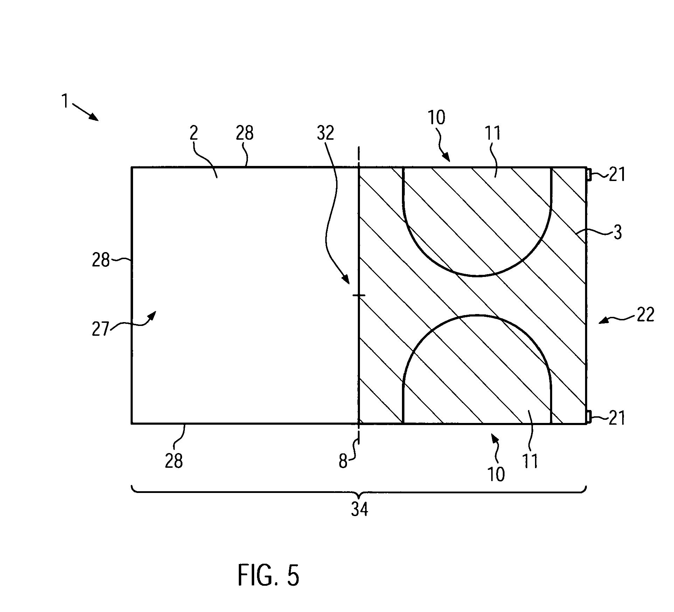

An access region 14 of the examination and therapy table surrounds or encircles the support region 3 of the examination and therapy table 1. Particularly, the access region 14 is the region next to the support region 3 into which the patient supporting plate 2 extends, as shown in Figure 3. Alternatively, the access region 14 can only be a limited access region 27, as indicated in Figure 5 and being encircled by table edge 28 and broken line 8. The limited access region 27 is beneath and above the respective therapy portion 7 or examination portion 6 that extends from one lateral side 35 of a supporting structure in the support region. It is to be understood that in the limited case, the projecting portion 6 or 7 of the patient supporting plate 2 may only be disposed extending from this one lateral side 35 of the support region 3.

The patient supporting plate 2 is formed as a carbon fiber part in which the therapy portion 7 and the examination portion 6 are integrally connected. In the current example, the patient supporting plate 2 is a one-piece plate comprising the examination portion 6 and the therapy portion 7.

In Figures 3 and 4, coordinate systems 30 and 31 are indicated and indicate directions to which it is referred. A "vertical" direction is approximately the Z direction, while the X-Y plane is also referred to as the approximately "horizontal" plane, having approximately "horizontal" X-Y directions.

The patient supporting plate 2 is rotatably connected to the supporting structure of table 1, which comprises the central column 4 and the X-Y positioning unit 5. The support structure of table 1 comprising column 4 and X-Y positioning unit 5 is capable of moving the patient supporting plate 2 in said X, Y and Z directions in order to undertake a fine-positioning of a patient under a medical appliance. Such a movement is in the range of centimeters and allows extending an imaging region in the horizontal plane by moving the patient supporting plate 2 and the patient lying thereon relative to the medical appliance. The therapy configuration or the examination configuration, respectively, is maintained during the operation of the X-Y positioning unit 5.

The central column 4 can be fixedly mounted to the ground, or can comprise projecting feet with rollers, so that the entire examination and therapy table can be mobile.

In order to carry out motions like the Trendelenburg rotation, the support structure is capable of rotating the patient supporting plate 2 about an axis of rotation, which extends approximately in the X direction, as shown in Figure 4. The location of such an axis of rotation approximately in the Y direction depends on the respective situation, and the table is capable of making a Trendelenburg movement with an isocentrical and non-isocentrical tilt feature.

The connection between the patient supporting plate 2 and the supporting structure is established in the support region by a shaft structure 15 in which a shaft 16 connected to the patient supporting plate 2 extends into a receiving bore 17. The receiving bore 17 is arranged extending through a mount 18, which laterally projects from the X-Y positioning unit 5 at least partially into the access region 14 of table 1. The shaft structure forms a center of rotation about which the patient supporting plate 2 can rotate relative to the support structure approximately about an axis in the Z direction. In this particular case, the shaft 16 forms a central axis 32 of the patient supporting plate 2 that is arranged approximately central relative to a width 33 and/or a length 34 of the patient supporting plate 2. The patient supporting plate 2 is rotatable by at least 180 degrees but could also be adapted to rotate freely, unless locked by the locking means 21.

The shaft structure renders an articulated joint that is capable of receiving transverse forces, said forces in approximately the X-Y plane, wherein mount 18 is capable of receiving normal forces in approximately the Z direction.

The patient supporting plate 2 comprises a first front end 19 adjacent the examination portion and a second front end 20 adjacent the therapy portion 7. A releasable locking means 21 is provided at a support front end 22 to lock the patient supporting plate 2 against a rotation about the center of rotation formed by shaft structure 15, and to compensate for torques that can cause the patient supporting plate 2 to rotate about an axis parallel to the delimiting broken line 8 and approximately in the X direction, as shown in Figures 1 and 4. Such torques can be induced by weight of a body lying on top of the patient supporting plate 2. Although the shaft structure 15 can be designed to support and compensate for said torques, the locking means 21 safely secures either the first front end 19 or the second front end 20 in place.

The locking means 21 can be designed to clamp down the respective front end 19 or 20, forming frontal sides, to the X-Y positioning unit. The locking means 21 engages the respective first or second front end 19 or 20 at respective mounting bores 23. Those mounting bores 23, which are arranged in the access region 14 of the table 1, depending on the actual configuration of the table 1, can serve as mounts for plate extensions 24, as indicated in Figure 1. Separate mounting bores 23 for both locking means 21 and plate extensions 24 could also be provided.

Locking means 21 and shaft structure 15 render two or three bearing means for supporting the patient supporting plate 2 on the table 1, the shaft structure 15 and the locking means 21 being arranged at opposing lateral sides of the X-Y positioning unit 5.

Mounting bores 23 could also be used to carry a leg support so that the table 1 assumes a gynecological examination configuration. Other appliances and accessories, like arm boards, shoulder supports and extensions, can either be attached to mounting bores 23 or to auxiliary mounting bores 25, which are disposed on lateral faces 26 of the patient supporting plate 2.

The use of the examination and therapy table 1 shall be described hereinafter.

During urogenital examination procedures, the therapy table 1 is usually converted into the examination configuration in which the examination portion 6 is arranged in the access region 14 of table 1. Radiography can be conducted without image interferences appearing on radiographic images due to the homogeneity of examination portion 6. The urogenital tract of a patient is positioned within the examination portion 6 for radiography while the head and thorax rest mainly on the therapy portion 7. A plate extension 24 can be attached to the second front end 20 of the patient supporting plate 2 in order to provide support for the patient's legs. Alternatively, the head and thorax of the patient can rest on the plate extension 24 while the patient's feet rest on the therapy portion 7. In order to provide a most comfortable underlay, inserts 11 close the cut-outs 10, thereby forming an approximately smooth top surface 29 of the patient supporting plate 2.

During respective therapy applications, image interferences caused by inhomogeneous portions of the table, such as cut-outs 10, are usually acceptable. However, during applications that focus on examination of a patient via radiography, image interferences should be avoided and the part of the patient that is to be x-rayed is placed in the examination portion 6 of the patient supporting plate 2. Therefore, the table 1 is converted from the examination configuration into the therapy configuration as needed.

In the therapy configuration, the therapy portion 7 is arranged in the access region 14 while the examination portion 6 is arranged in the support region 3 of table 1. One or two of the inserts 11 are removed from the patient supporting plate 2 in order to clear the cut-outs 10. A lithotripter having a C-arm can now easily access the respective body portions of the patient that are arranged in the therapy portion 7 of the patient supporting plate 2. Radiography can be conducted simultaneously.

In order to switch between both configurations, the locking means 21 simply needs to be released from its engagement with mounting bores 23 on the respective front ends 19 or 20. The patient supporting plate 2 can then easily be rotated about an approximately vertically extending axis of rotation defined by shaft structure 15.

As soon as either the examination portion 6 or the therapy portion 7 is transferred to the support region 3, the locking means 21 can be reconnected to engage the mounting bores 23. If a plate extension 24 was attached to patient supporting plate 2 prior to conversion, it can be reconnected to the respective front end 19 or 20 once the locking means 21 engages the respective mounting bores 23.

## Claims

1. Examination and therapy table (1) having
an access region (14),
a support region (3), and
a patient supporting plate (2),
the patient supporting plate (2) comprising:
an examination portion (6) having means for facilitating examination and a therapy portion (7) having means for facilitating therapy,
wherein there is an examination configuration in which the examination portion (6) of the patient supporting plate (2) is arranged in the access region (14) of the table (1), whereas the therapy portion (7) of the patient supporting plate (2) is arranged in the support region (3) of the table (1),
**characterized in that**
the table (1) is convertible into a therapy configuration, in which the therapy portion (7) of the patient supporting plate (2) is arranged in the access region (14) of the table (1) and the examination portion (6) of the patient supporting plate (2) is arranged in the support region (3) of the table (1).

2. Examination and therapy table (1) according to claim 1,
**characterized in that**
the examination portion (6) is moveable in conjunction with the therapy portion (7) from the examination configuration to the therapy configuration and vice versa.

3. Examination and therapy table (1) according to one of the preceding claims, **characterized in that**
the access region (14, 27) adjoins one lateral side of the support region (3).

4. Examination and therapy table (1) according to one of the preceding claims, **characterized in that**
the patient supporting plate (2) is rotatable between the examination configuration and the therapy configuration.

5. Examination and therapy table (1) according to claim 4,
**characterized in that**
a center of rotation is arranged approximately at a transition region (9) between the access region (14) and the support region (3) of the examination and therapy table (1).

6. Examination and therapy table (1) according to one of the preceding claims, **characterized in that**
the patient supporting plate (2) is rotatably connected to a supporting means (4) of the examination and therapy table (1).

7. Examination and therapy table (1) according to one of the preceding claims, **characterized in that**
the patient supporting plate (2) is rotatable about a central axis (32) arranged approximately central relative to a width (33) and/or a length (34) of the patient supporting plate (2).

8. Examination and therapy table (1) according to one of the preceding claims, **characterized in that**
the patient supporting plate (2) is connected to a supporting means (4) via an articulated joint, the articulated joint being capable of receiving transverse forces approximately in X-Y directions and normal forces in an approximately Z direction.

9. Examination and therapy table (1) according to one of the preceding claims, **characterized in that**
the patient supporting plate (2) is connected to a supporting means (4) of the examination and therapy table (1) via a shaft structure (15).

10. Examination and therapy table (1) according to one of the preceding claims, **characterized in that**
at least one locking means (21) is provided, which locks the patient supporting plate (2) in at least the examination configuration and/or the therapy configuration, preferably against a rotation.

11. Examination and therapy table (1) according to claim 10,
**characterized in that**
the locking means (21) is releasable.

12. Examination and therapy table (1) according to one of claims 10 or 11, **characterized in that**
the locking means (21) comprises means for securing the patient supporting plate (2) against rotation about an axis (32) approximately in the Z direction.

13. Examination and therapy table (1) according to one of the preceding claims, **characterized in that**
the patient supporting plate (2) integrally incorporates at least the examination portion (6) and the therapy portion (7).

14. Examination and therapy table (1) according to one of the preceding claims, **characterized in that**
the examination portion (6) is homogeneous.

15. Examination and therapy table (1) according to one of the preceding claims, **characterized in that**
the therapy portion (7) of the patient supporting plate (2) is permeable for radiography.
